# EUROPÄISCHE PATENTSCHRIFT

(11) **EP 1 668 352 B1**
(45) Veröffentlichungstag und Bekanntmachung des Hinweises auf die Patenterteilung: **18.12.2013**
(21) Anmeldenummer: 04763222.9
(22) Anmeldetag: 15.07.2004
(51) Int. Cl.: G01N 29/02, A61B 8/06

(54) **Akustisches Verfahren zum Messen einer Signallaufzeit in Blut und Vorrichtung zur Anwendung des Verfahrens**
Acoustic method for measuring a signal propagation time in blood and device for using this method
Procédé acoustique pour mesurer le temps de propagation d'un signal dans du sang et dispositif pour appliquer ce procédé

(30) Priorität: 22.08.2003 DE 10338940
(43) Veröffentlichungstag der Anmeldung: 14.06.2006
(73) Patentinhaber: Fresenius Medical Care Deutschland GmbH, 61352 Bad Homburg v.d.H. (DE)
(72) Erfinder: KOERDT, Franz-Wilhelm, 61231 Bad Nauheim (DE); SCHEUNERT, Peter, 61381 Friedrichsdorf (DE); NIKOLIC, Dejan, 60599 Frankfurt (DE); METZNER, Klaus, 61381 Friedrichsdorf (DE); KÜHN, Alexander, 36341 Lauterbach (DE)
(74) Vertreter: Ziermann, Oliver
(86) Internationale Anmeldenummer: PCT/EP2004/007812
(87) Internationale Veröffentlichungsnummer: WO 2005/029064

(56) Entgegenhaltungen:
- EP-A- 0 855 577
- EP-A- 1 077 365
- DE-A1- 19 809 945
- DE-C- 10 106 308
- US-A- 4 028 938
- US-A- 4 754 650
- US-A- 5 123 286
- US-B1- 6 226 598
- US-B1- 6 542 761

## Beschreibung

Die Erfindung betrifft das Gebiet der Signallaufzeitsensorik, insbesondere der Sensorik auf der Basis von Ultraschalllaufzeiten.

Die Ausbreitungsgeschwindigkeit eines Signales innerhalb eines Mediums ist abhängig von der Zusammensetzung des Mediums. Auf diese Weise ist durch Messung der Ausbreitungsgeschwindigkeit eine Schlussfolgerung auf das Medium selbst möglich. Die entsprechenden Messverfahren basieren dabei oft auf der Ausbreitung von Ultraschall. Das Messobjekt, das z.B. in Form einer fluidführenden Leitung mit dem zu untersuchenden Fluid vorliegen kann, wird dabei innerhalb einer Messstrecke angeordnet, die einen Ultraschallsender von einem Ultraschallempfänger trennt. Wenn die Länge der Messstrecke bekannt ist, kann mit Hilfe der Signallaufzeit vom Sender zum Empfänger die Ausbreitungsgeschwindigkeit bestimmt werden. Ist nur eine relative Änderung von Interesse, so kann aus der relativen Änderung der Signallaufzeit direkt auf die relative Änderung der Ausbreitungsgeschwindigkeit geschlossen werden, solange sich die Messstrecke nicht in unbekanntem Maße ändert.

Die Messstrecke kann dabei in verschiedene Bereiche aufgeteilt sein. Durchfließt ein Medium eine Leitung, so setzt sich die Messstrecke aus einem ersten Bereich, der die Wandungen der Leitungen umfasst, und einem zweiten Bereich, der das eigentliche Messmedium durchquert, zusammen. Ändert sich nun die Zusammensetzung des Mediums, so ist die sich ergebende Änderung der Signallaufzeit im Allgemeinen allein auf die Änderung der Signallaufzeit im zweiten Bereich zurückzuführen, da sich die Signallaufzeit im ersten Bereich bei geeigneter Wahl der Wandungen nicht ändert. Ist die Signallaufzeit in dem ersten Bereich aufgrund der Kenntnis des Wandungsmaterials und seiner Abmessungen bekannt, läßt sich auch leicht die auf den zweiten Bereich entfallende absolute Signallaufzeit bestimmen.

Bei der Hämodialysebehandlung wird Blut in einem extrakorporalen Blutkreislauf kontinuierlich von einem Patienten entfernt, durch einen Hämodialysator gereinigt und an den Patienten zurückgegeben. Als Nierenersatzbehandlung ist dabei die gleichzeitige Entfernung von nicht abgeschiedener Flüssigkeit erforderlich. Hierbei wird dem Patienten während der Behandlung meist eine vorgegebene Menge an Flüssigkeit während der Behandlung entzogen. Durch einen zu raschen Flüssigkeitsentzug kann es jedoch zu unerwünschten Begleiterscheinungen wie einen zu großen Blutdruckabfall - der Hypotonie - kommen.

Da die durch den Flüssigkeitsentzug verursachte Blutvolumenverringerung zu einer steigenden Blutdichte bzw. einem fallenden Blutwasseranteil führt, der sich im Allgemeinen auch in einem steigenden Hämatokrit manifestiert, ist die Überwachung des extrakorporalen Blutes mit Hilfe eines Ultraschalllaufzeitsensors in der US 5,230,341 vorgeschlagen worden. In der DE 100 51 943 A1 wird der Einfluss von Blutdichteschwankungen auf Pulswellenlaufzeitmessungen behandelt, wobei in einer Ausführungsform Ultraschalllaufzeitmessungen zur Erfassung der Blutdichte vorgesehen sind.

Ultraschalllaufzeitmessungen sind in der DE 198 09 945 A1 auch zur Überwachung der Zusammensetzung der Dialysierflüssigkeit vorgeschlagen worden. Die Dämpfung von Ultraschallsignalen wird, wie z.B. in der EP 0 899 564 A2 beschrieben, zur Erkennung von Luftblasen bei der Infusion von Flüssigkeiten in einen Patienten ausgewertet.

Zur Steuerung und Auswertung derartiger Sensoren werden bestimmte elektronische Schaltungen eingesetzt, um die erforderliche Genauigkeit zu gewährleisten. Ein Beispiel einer solchen Schaltung ist in DE 34 20 794 C2 beschrieben.

Ein multifunktioneller Ultraschalllaufzeitsensor für die Messung an extrakorporalem Blut, bei dem ein preiswertes Wegwerfteil für die Leitung des Blutes durch die Messstrecke eingesetzt werden kann, ist Gegenstand der US 6,542,761.

Auf dem Gebiet der Sensorik an Erdgasleitungen beschreibt die EP 0 902 883 B1 ein Verfahren, mit dem ein Nullpunktdurchgang eines Schallempfangssignals zur Fortpflanzungszeitmessung eines Schallsignales bestimmt wird.

Bestehende elektronische Steuerschaltungen für Laufzeitsensoren in Blut sind extrem aufwendig oder in ihrer zeitlichen Auflösung begrenzt. Der Erfindung liegt daher die Aufgabe zu Grunde, ein einfaches und wenig anfälliges Laufzeitmessverfahren zur Anwendung an Blut, bei gleichzeitig hoher zeitlicher Auflösung bereitzustellen. Der Erfindung liegt auch die Aufgabe zu Grunde, eine Vorrichtung zur Anwendung dieses Verfahrens bereitzustellen.

Nach der Lehre der Erfindung wird diese Aufgabe durch ein Verfahren zum Messen einer Signallaufzeit oder einer Signallaufzeitänderung mit den Merkmalen des Anspruchs 1 und durch eine Vorrichtung zur Anwendung des Verfahrens mit den Merkmalen des Anspruchs 9 gelöst. Vorteilhafte Ausgestaltungen der Erfindung sind Gegenstand der Unteransprüche.

Das erfindungsgemäße Verfahren bedient sich dabei einer einfachen Messtechnik, wie sie für die zu erreichende Zeitauflösung zunächst nicht direkt verwendet werden kann. Dabei wird ein gewöhnliches Abtastverfahren ("Sampling") zur Erfassung des Empfangssignals eingesetzt. Da das Messmedium bei den hier relevanten Zeitbereichen im Nano- und Subnanosekundenbereich bereits die Wirkung eines Tiefpasses sowie der Empfänger die Ausbildung von Resonanzfrequenzen zeigt, verursacht ein abgestrahltes stufenartiges Signal ein schwingungsartiges Empfangssignal. Erfindungsgemäß wird dieses Empfangssignal zumindest während einer Halbperiode abgetastet und mit Hilfe eines Auswahlkriteriums überprüft. Nur bei positiver Überprüfung wird mindestens ein inter- oder extrapolierter Berührungspunkt des Empfangssignals mit einem Ruhepegel in einem Empfangssignal-Zeit-Diagramm bestimmt, mit deren Hilfe die Signallaufzeit oder zumindest die Signallaufzeitänderung ermittelt wird.

Das Auswahlkriterium garantiert dabei eine verlässliche Erkennung eines Empfangssignals als Antwort auf ein abgestrahltes Signal. Der nachgehende Inter-oder Extrapolationsschritt führt zu einer beachtlichen Erhöhung der zeitlichen Auflösung.

Als Auswahlkriterium können unterschiedliche Bedingungen herangezogen werden. Hier kann die zwischen dem Ruhepegel und dem Empfangssignal während der Halbperiode eingeschlossene Fläche mit Vergleichswerten verglichen werden. Auch kann eine weitere, nachfolgende Halbperiode entsprechend ausgewertet werden. Schließlich muss für die Auswertung nicht die erste Halbperiode herangezogen werden. Irgendeine ausgewählte Halbperiode ist ausreichend.

Schließlich können durch entsprechende Fertigungsvorgaben die Resonanzfrequenzen des Empfängers sehr genau vorgegeben sein, so dass die Zeitdauer der Halbperiode ebenfalls als Auswahlkriterium herangezogen werden kann. Hierbei ist allerdings zu beachten, dass aufgrund von Überlagerungseffekten nicht jede Halbperiode die gleiche Zeit andauert. Es ist vielmehr so, dass jede Halbperiode für sich eine genau definierte Zeitdauer aufweist.

In vorteilhafter Ausführungsform der Erfindung wird aus dem erfassten Empfangssignal auch die Dämpfung des Signals ermittelt, so dass die erfindungsgemäße Vorrichtung auch geeignet ist, neben der Zusammensetzung eines Mediums auch Einschlüsse wie z.B. Luftblasen in Blut zu erkennen, die mit einer Dämpfung des Signales einhergehen.

Weitere Einzelheiten und Vorteile der Erfindung werden anhand eines in den Zeichnungen dargestellten Ausführungsbeispiels näher beschrieben. Es zeigen:
Fig. 1 eine schematische Darstellung einer Ausführungsform der erfindungsgemäßen Vorrichtung,
Fig. 2 des abgestrahlte und Empfangssignal als Funktion der Zeit und
Fig. 3 einen vergrößerten Ausschnitt des Empfangssignal-Zeit-Diagamms von Fig. 2.

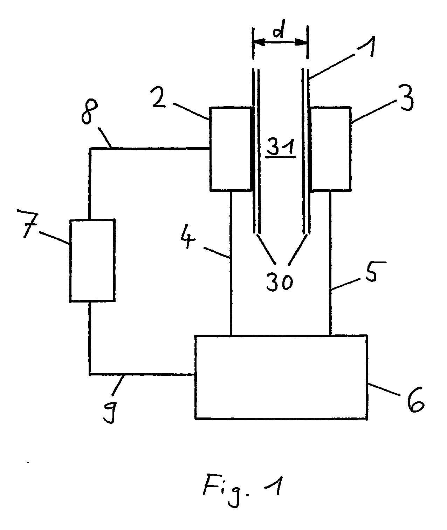

In Fig. 1 ist eine Ausführungsform der erfindungsgemäßen Vorrichtung zur Anwendung des erfindungsgemäßen Verfahrens schematisch dargestellt. Eine blutführende Leitung 1 eines befindet sich dabei zwischen einem Ultraschallsender 2 und einem Ultraschallempfänger 3, die durch eine Messstrecke d voneinander beabstandet sind. Die Messstrecke d ist dabei in zwei Bereiche 30 und 31 geteilt, wobei der erste Bereich 30 auf die Wandungen der Leitung 1 und der zweite Bereich auf den vom Blut durchflossenen Bereich 31 entfällt. Bei der Leitung 1 kann es sich z.B. um ein folienartiges Wegwerfteil wie in der US 6,542,761 beschrieben handeln.

Des Weiteren ist eine Auswerteeinheit 6 vorgesehen, die über eine Signalleitung 4 mit dem Ultraschallsender 2 und über eine Signalleitung 5 mit dem Ultraschallempfänger 3 verbunden ist. Sowohl die Auswerteeinheit 6 als auch der Ultraschallsender 2 werden über Systemleitungen 8 und 9 durch einen Oszillator 7 mit einem Systemtakt versorgt.

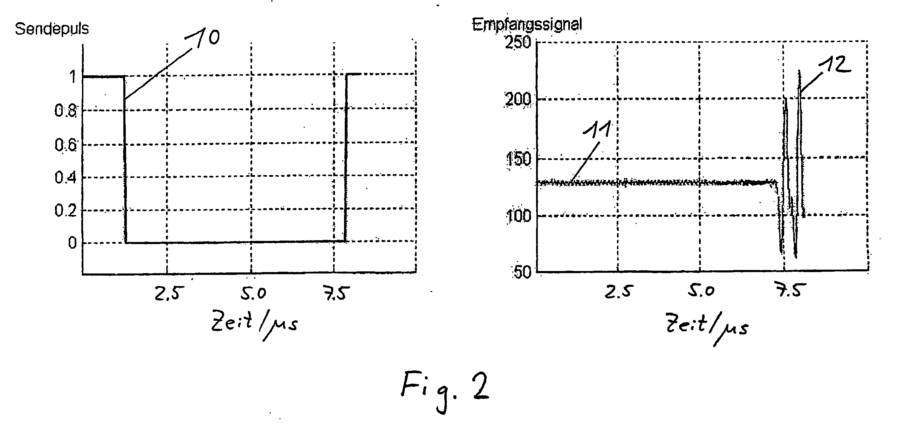

Der Ultraschallsender 2 sendet stufenartige Ultraschallsignale 10 (Fig. 2) sowie synchronisierte Signale über die Signalleitung 4 an die Auswerteeinheit 6. An dem Ultraschallempfänger 3 stellt sich als Antwort auf das stufenartige Signal 10 ein um einen Ruhepegel 11 schwingungsartiges Empfangssignal 12 ein, das über die Signalleitung 5 ebenfalls an die Auswerteeinheit 6 weitergegeben wird. Es ist dabei auch möglich, dass anstelle der abfallenden Stufe die nachsteigende ansteigende Stufe für die Anwendung des erfindungsgemäßen Verfahrens angewendet wird.

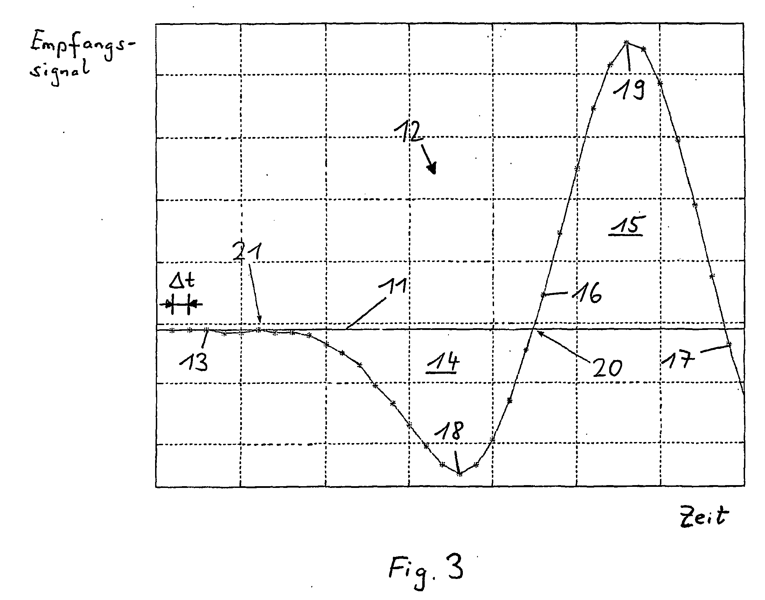

In Fig. 3 ist das schwingungsartige Empfangssignal 12 von Fig. 2 vergrößert dargestellt. Dabei sind die einzelnen Signalwerte 13 dargestellt, die die Auswerteeinheit 6 als Wert eines mit der Signalleitung 5 verbundenen A/D-Wandlers in regelmäßigen Zeitabständen Δt abtastet und hinterlegt. Die durch einen als temperaturkompensierten Quarzoszillator ausgeführten Oszillator 7 vorgegebene Abtastrate beträgt dabei typischerweise f=80 MHz, d.h. Δt=1/f=12,5 ns. Zur Erhöhung des Signal-Rausch Verhältnisses kann das Empfangssignal über einen gleitende Mittelwertbildung geglättet bzw. gefiltert werden.

Zur Erkennung eines von dem abgestrahlten stufenartigen Signal 10 verursachten schwingungsartigen Empfangssignales 12 werden die hinterlegten Signalwerte 13 durch ein Auswahlkriterium überprüft. Hierzu wird erfindungsgemäß mindestens eine Halbperiode 14 des Empfangssignales abgetastet, die in Fig. 3 bis zum Signalwert 16 reicht. Besonders vorteilhaft ist die Erfassung auch der sich anschließenden Halbperiode 15 bis zum Signalwert 17.

Zunächst ermittelt die Auswerteeinheit 6 den Ruhepegel 11. Hierzu kann eine fortschreitende Mittelwertbildung bis zu einem Abbruchkriterium eingesetzt werden. So kann z.B. ein Unterschreiten um einen vorgegebenen Wert oder ein wiederholtes Unterschreiten des bisher ermittelten Ruhepegels den Beginn der Halbperiode 14 und ein Abbruch der Mittelwertbildung für den Ruhepegel 11 bedeuten. Tritt dieses Ereignis ein, integriert die Auswerteeinheit 6 die zwischen dem Ruhepegel 11 und der durch die Signalwerte 13 eingeschlossene Fläche während der Halbperiode 14. Das Ende der Integration wird durch Überschreiten des Ruhepegels 11 durch den Signalwert 16 erkannt. Im Allgemeinen ist es bei der Integration im Sinne des Auswahlkriteriums ausreichend, die eingeschlossene Fläche als Summe der um den Ruhepegel reduzierten Signalwerte der Halbperiode zu ermitteln. Eine Multiplikation mit der konstanten Abtastperiode Δt erwirkt nur ein proportionales Ergebnis. Für die Steigerung der Genauigkeit kann jedoch bei Bedarf auch auf interpolierte Kurven, insbesondere Geraden, zwischen den Signalwerten 13 zurückgegriffen werden. Dies geht jedoch mit einer beträchtlichen Erhöhung des Rechenaufwandes einher.

Weiterhin ermittelt die Auswerteeinheit 6 die Anzahl der die Halbperiode umfassenden Signalwerte 13 und damit die Zeitdauer der Halbperiode 14 sowie in einer vorteilhaften Ausführungsform zusätzlich den Extremwert 18 der Halbperiode 14. Für alle diese Größen sind in der Auswerteeinheit 6 Vergleichswerte hinterlegt, die mit den gemessenen Werten verglichen werden. Endet der Vergleich im Sinne des Auswahlkriteriums positiv, wird das schwingungsartige Empfangssignal 12 als für eine Messung der Signallaufzeit oder der Signallaufzeitänderung zu verwertendes Signal erkannt. Andernfalls verwirft die Auswerteeinheit 6 diesen Messtakt.

Bezogen auf die Zeitdauer der Halbperiode 14 ist anzumerken, dass diese aufgrund des Resonanzverhaltens des meist als Piezokristall ausgeführten Ultraschallempfängers 3, das zudem durch seine Geometrie genau vorgegeben sein kann, innerhalb genau definierter Grenzen liegen muss - unabhängig von der eigentlichen Laufzeit des Signales. Dieses Auswahlkriterium stellt daher ein sehr gut diskriminierendes Kriterium dar.

Es ist auch denkbar, dass in der Auswerteeinheit 6 nur einzelne der genannten Auswahlkriterien angewendet werden. In der Praxis hat sich dabei eine Kombination der Auswertung der Extremstelle sowie der Zeitdauer der Halbperiode bewährt.

Besonders vorteilhaft ist die entsprechende Auswertung des schwingungsartige Empfangssignals 12 auch noch während der sich anschließenden Halbperiode 15 bis zum Signalwert 17. Hier können neben der Fläche der Halbperiode 15, dem Extremwert 19 oder der Zeitdauer der Halbperiode 15 auch Beziehungen unterhalb der einzelnen Parameter wie z.B. das Verhältnis der um den Ruhepegel reduzierten Extremwerte 18 und 19 oder der Flächen der Halbperioden 14 und 15 einem Auswahlkriterium unterzogen werden. Auf diese Weise kann die Zuverlässigkeit der Auswahl eines einem abgestrahlten stufenartigen Signal zuzuordnenden Empfangssignals zunehmend gesteigert werden.

Nach positiver Überprüfung des Empfangssignals bestimmt die Auswerteeinheit 6 die Signallaufzeit oder die Signallaufzeitänderung. Hierzu ermittelt die Auswerteeinheit 6 einen inter- oder extrapolierten Berührungspunkt des schwingungsartigen Empfangssignals 12 mit dem Ruhepegel 11 in dem Empfangssignal-Zeit-Diagramm. Sehr genaue Ergebnisse lassen sich durch Ermittlung des interpolierten Berührungspunktes 20 zwischen der ersten und zweiten Halbperiode 14 und 15 erreichen. Da hier ein Empfangssignal, bei dem die Überprüfung mit dem Auswahlkriterium positiv verlaufen ist, eine ausreichend steile Steigung hat, kann dieser Berührungspunkt 20 mit hoher Genauigkeit durch Interpolation mit Hilfe einer die beiden benachbarten Signalwerte durchlaufenden Geraden ermittelt werden. Es ist auch möglich, andere Kurvenformen und weitere benachbarte Signalwerte für die Interpolation heranzuziehen. Dem Fachmann sind hierfür hinreichende Mittel und Verfahren geläufig.

Aus dem zeitlichen Abstand zwischen dem abgestrahlten stufenartigen Signal 10 und der Zeitkoordinate des Berührungspunktes 20 ermittelt die Auswerteeinheit einen Wert für die Signallaufzeit. Diese stellt jedoch nicht die absolute Signallaufzeit dar, welche vielmehr durch den Berührungspunkt 21 am Beginn der ersten Halbperiode 14 markiert wird. Die Bestimmung des Berührungspunktes 21 durch einen ähnlichen Interpolationsvorgang ermöglicht die Bestimmung der absoluten Signallaufzeit. Da die Steigung an dieser Stelle weniger steil verläuft, erreicht der ermittelte Wert jedoch im Allgemeinen nicht die Genauigkeit, mit der Berührungspunkt 20 bestimmt werden kann. Auch sind hier andere Kriterien zur Auswahl der zu berücksichtigenden Signalwerte 13 anzuwenden, da sich am Beginn der Halbperiode 14 nicht unbedingt genau ein Signalwert über und genau ein benachbarter Signalwert unter dem Ruhepegel 11 befinden. In diesem Fall kann es sich anbieten, den Berührungspunkt 21 durch Extrapolation des Kurvenverlaufes durch die ersten Signalwerte 13 der ersten Halbperiode 14 zu bestimmten, wobei je nach Kurvenform Geraden oder andere Funktionen verwendet werden können.

Die Möglichkeit der Extrapolation besteht natürlich auch für andere Berührungspunkte wie den Berührungspunkt 20. Hier wird das Verfahren der Interpolation jedoch im Allgemeinen vorzuziehen sein.

Aufgrund des Resonanzverhaltens des Ultraschallempfängers 3 ist jedoch der zeitliche Abstand der Berührungspunkte 20 und 21 unabhängig von der Signallaufzeit konstant. Mit anderen Worten bedeutet dies, dass sich eine Änderung der Signallaufzeit durch eine Änderung der Blutzusammensetzung im Bereich 31 der Leitung 1 zwischen zwei Zeitpunkten t₁ und t₂ bei beiden Punkten gleichermaßen auswirkt. Eine entsprechende Signallaufzeitänderung lässt sich also mit Hilfe des Berührungspunktes 20 sehr genau bestimmen. Obwohl der zeitliche Abstand der Signalwerte 13 bei Δt=12,5 ns liegt, können damit aufgrund des erfindungsgemäßen Verfahrens Zeitauflösungen im Subnanosekundenbereich erreicht werden. Auch die Ermittlung der Zeitkoordinate des Berührungspunkts 21 zur absoluten Laufzeitmessung erlaubt zumindest eine deutliche Steigerung der Genauigkeit.

Schließlich kann bei sehr konstanten Versuchsbedingungen die absolute Signallaufzeit auch durch Ermittlung des Berührungspunktes 20 und durch Subtraktion der Zeitdauer der ersten Halbperiode 14, die als vorbekannter Wert für diese Versuchsbedingungen in der Auswerteeinheit 6 hinterlegt ist, bestimmt werden.

Mit Hilfe der bestimmten Signallaufzeit oder der Signallaufzeitänderung kann die Auswerteeinheit 6 aufgrund hinterlegter Informationen die Zusammensetzung des die Leitung 1 durchfließenden Blutes hier den Blutwassergehalt des die Leitung durchfließenden Blutes ermitteln. Dabei kann auch eine relative Angabe ausreichend sein, die eine Veränderung des Blutwassergehaltes oder der Blutdichte und damit des Blutvolumens relativ zu einem Anfangswert am Beginn der Messungen angibt. Dabei kann der Sensor an dem extrakorporalen Kreislauf eines Hämodialysegerätes angeordnet sein, um die Bestimmung der Veränderung des Blutvolumens während einer Hämodialysebehandlung zu erlauben.

Gleichzeitig kann die Auswerteeinheit 6 die Laufzeitmessung dazu verwenden, dass die Leitung 1 durchfließende Medium zu diskriminieren. So wird ein extrakorporaler Kreislauf am Beginn einer Hämodialysebehandlung mit isotoner Kochsalzlösung vorgefüllt und am Ende einer Behandlung gespült. Da die Laufzeiten in Blut und in Kochsalzlösung fundamental verschieden sind, kann die Auswerteeinheit 6 das Vorhandensein der einen oder anderen Flüssigkeit in der Leitung 1 unterscheiden, was zur Statusüberwachung oder Steuerung des Hämodialysegerätes ausgenutzt werden kann. Zweckmäßigerweise ist die Auswerteeinheit 6 in diesem Fall Teil der ohnehin in Hämodialysegeräten vorgesehenen Auswerte- und/oder Steuereinheit.

Ferner kann die Auswerteeinheit 6 die ermittelten Flächen der Halbperioden 14 und/oder 15 als Maß für die Signaldämpfung auswerten. Auf diese Weise kann die erfindungsgemäße Vorrichtung auch als Lufterkennungssensor eingesetzt werden, da bereits kleine Einschlüsse in Form von Luftblasen in dem die Leitung 1 durchfließenden Blut zu einer Verminderung des Signals am Empfänger 3 führen. Befindet sich nur Luft in der Leitung 1, ist dieser Effekt zusätzlich zu der Laufzeitänderung besonders ausgeprägt. Die Auswerteeinheit 6 ist in dieser besonders vorteilhaften Ausführungsform geeignet, entsprechende Alarmsignale an das Hämodialysegerät zu geben, damit eine den Patienten gefährdende Infusion von Luft unterbunden werden kann.

Die Erfindung stellt ein Verfahren und eine Vorrichtung bereit, mit der trotz einfacher Messkomponenten eine verlässliche Messung unter hoher Zeitauflösung von Signallaufzeiten oder Änderungen von Signallaufzeiten ermöglicht wird. Die Erfindung findet Anwendung zur Bestimmung der Zusammensetzung von Blut in einem extrakorporalen Blutkreislauf während einer Blutbehandlung wie der Hämodialyse, bei der eine Beobachtung der Veränderung des Blutvolumens zur Vermeidung von unerwünschten Nebeneffekten angestrebt wird. Gleichzeitig kann der erfindungsgemäße Sensor als Lufterkennungssensor eingesetzt werden.

## Patentansprüche

1. Verfahren zum Messen einer Zusammensetzung bzw. einer Zusammensetzungsänderung von Blut durch Messen einer Signallaufzeit in Blut, die ein Signal zum Durchlaufen einer Messstrecke von einem Ultraschallsender (2) zu einem Ultraschallempfänger (3) benötigt, wobei sich eine blutführende Leitung in der Messstrecke befindet, oder von Änderungen dieser Signallaufzeit, wobei
mit dem Ultraschallsender (2) ein stufenartiges Signal (10) abgestrahlt wird und
das stufenartige Signal (10) nach Durchlaufen der Messstrecke zu einem schwingungsartigen Empfangssignal (12) um einen Ruhepegel (11) an dem Ultraschallempfänger (3) führt, das in regelmäßigen Zeitabständen Δt abgetastet und erfasst wird,
dass das schwingungsartige Empfangssignal (12) anhand eines Auswahlkriteriums zumindest während einer Halbperiode (14, 15) dahingehend überprüft wird, ob es sich um das vom stufenartigen Signal (10) verursachte Empfangssignal handelt und
dass bei positiver Überprüfung die Signallaufzeit oder die Signallaufzeitänderung mit Hilfe eines inter- oder extrapolierten Berührungspunktes (20, 21) des schwingungsartigen Empfangssignals (12) mit dem Ruhepegel (11) in einem Empfangssignal-Zeit-Diagramm ermittelt wird, und die Signallaufzeit bzw. die Signallaufzeitänderung als Maß für die Zusammensetzung bzw. die Zusammensetzungsänderung des Blutes aufgrund hinterlegter Informationen ausgewertet wird, wobei die zwischen dem schwingungsartigen Empfangssignal (12) und dem Ruhepegel (11) eingeschlossene Fläche während der Halbperiode (14) ermittelt wird und die so ermittelte Fläche als Auswahlkriterium mit einem Vergleichswert verglichen wird, oder als Auswahlkriterium die Zeitdauer einer oder mehrerer Halbperioden (14, 15) des schwingungsartigen Empfangssignals (12) ermittelt und mit einem Vergleichswert verglichen wird.

2. Verfahren nach Anspruch 1, **dadurch gekennzeichnet, dass** als inter- oder extrapolierter Berührungspunkt der Punkt (21) im Empfangssignal-Zeit-Diagramm ermittelt wird, bei dem das schwingungsartige Empfangssignal (12) am Beginn der ersten Halbperiode (14) vom Ruhepegel (11) abweicht, wobei aus dem so ermittelten Zeitpunkt die Signallaufzeit abgeleitet wird.

3. Verfahren nach Anspruch 1, **dadurch gekennzeichnet, dass** als inter- oder extrapolierter Berührungspunkt der Punkt (20) im Empfangssignal-Zeit-Diagramm ermittelt wird, bei dem das schwingungsartige Empfangssignal (12) den Ruhepegel (11) nach der ersten Halbperiode (14) schneidet, wobei aus dem so ermittelten Zeitpunkt die Signallaufzeitänderung abgeleitet wird.

4. Verfahren nach einem der vorhergehenden Ansprüche, **dadurch gekennzeichnet, dass** die zwischen dem schwingungsartigen Empfangssignal (12) und dem Ruhepegel (11) eingeschlossen Fläche während der Halbperiode (14) ermittelt wird, dass auch die anschließende Halbperiode (15) abgetastet, erfasst und die zwischen dem schwingungsartigen Empfangssignal (12) und dem Ruhepegel (11) eingeschlossene Fläche während der anschließenden Halbperiode (15) ermittelt wird und, dass die zwischen dem schwingungsartigen Empfangssignal (12) und dem Ruhepegel (11) eingeschlossene Fläche während der anschließenden Halbperiode (15) als Auswahlkriterium mit einem Vergleichswert verglichen wird.

5. Verfahren nach einem der Ansprüche 1 bis 3, **dadurch gekennzeichnet, dass** der Extremwert (18) des schwingungsartigen Empfangssignals (12) während der Halbperiode (14) ermittelt und mit einem Vergleichswert verglichen wird.

6. Verfahren nach Anspruch 5, **dadurch gekennzeichnet, dass** auch die anschließende Halbperiode (15) abgetastet und erfasst wird und als weiteres Auswahlkriterium der Extremwert (19) des schwingungsartigen Empfangssignals (12) während der anschließenden Halbperiode (15) ermittelt und mit einem Vergleichswert verglichen wird.

7. Verfahren nach einem der vorhergehenden Ansprüche, **dadurch gekennzeichnet, dass** der Ruhepegel (11) als Mittelwert aus der Halbperiode (14) vorangegangenen, abgetasteten Empfangssignalwerten (13) ermittelt wird.

8. Verfahren nach Anspruch 4, **dadurch gekennzeichnet, dass** die ermittelten Flächen als Maß für die Dämpfung des Signals ausgewertet werden.

9. Vorrichtung zur Anwendung des Verfahrens nach einem der Ansprüche 1 bis ,8 mit
einem Ultraschallsender (2) zum Abstrahlen des stufenartiges Signales (10),
einem von dem Ultraschallsender (2) durch die Messstrecke beabstandeten Ultraschallempfängers (3) zum Abgeben eines um einen Ruhepegel (11) schwingungsartigen Empfangssignales (12) als Antwort auf das die Messstrecke durchlaufenden stufenartigen Signales (10),
einer sich in der Messstrecke befindenden blutführende Leitung (1),
einer mit dem Ultraschallsender (2) und dem Ultraschallempfänger (3) verbundenen Auswerteeinheit (6),
wobei die Auswerteeinheit (6) zum Absenden des Sendesignals synchrone Signale erhält und über eine Abtasteinrichtung zum Abtasten und Hinterlegen des schwingungsartigen Empfangssignales (12) in regelmäßigen Zeitabständen Δt verfügt,
wobei die Auswerteeinheit (6) ferner eingerichtet ist, das schwingungsartige Empfangssignal (12) anhand eines Auswahlkriteriums zumindest während einer Halbperiode (14, 15) dahingehend zu überprüfen, ob es sich um das vom stufenartigen Signal (10) verursachte Empfangssignal handelt, wobei die zwischen dem schwingungsartigten Empfangssignal (12) und dem Ruhepegel (11) eingeschlossene Fläche während der Halbperiode (14) ermittelt wird und die so ermittelte Fläche als Auswahlkriterium mit einem Vergleichswert verglichen wird, oder als Auswahlkriterium die Zeitdauer einer oder mehrerer Halbperioden (14, 15) des schwingungsartigen Empfangssignals (12) ermittelt und mit einem Vergleichswert verglichen wird und
bei positiver Überprüfung die Signallaufzeit oder die Signallaufzeitänderung mit Hilfe eines inter- oder extrapolierten Berührungspunktes (20, 21) des schwingungsartigen Empfangssignals (12) mit dem Ruhepegel (11) in einem Empfangssignal-Zeit-Diagramm zu ermitteln, und wobei die Auswerteeinheit (6) ferner geeignet ist, die Signallaufzeit bzw. die Signallaufzeitänderung als Maß für die Zusammensetzung bzw. die Zusammensetzungsänderung des Blutes aufgrund hinterlegter Informationen auszuwerten.

10. Vorrichtung nach Anspruch 9, **dadurch gekennzeichnet, dass** es sich um einen Blutvolumensensor handelt.

11. Vorrichtung nach Anspruch 10, **dadurch gekennzeichnet, dass** es sich ferner um einen Lufterkennungssensor handelt.

## Claims

1. A method for measuring a blood composition and/or a change in a blood composition by measuring a signal propagation time in blood, which requires a signal to propagate from an ultrasonic transmitter (2) to an ultrasonic receiver (3) through a measuring zone, such that a line carrying blood is situated in the measuring zone, or for measuring changes in said signal propagation time, wherein
a step-like signal (10) is emitted by the ultrasonic transmitter (2) and
after propagation of the step-like signal (10) through the measuring zone, it leads to a vibrational received signal (12) around a quiescent level (11) on the ultrasonic receiver (3), said received signal being scanned and detected at regular intervals in time Δt,
the vibrational received signal (12) is checked on the basis of a selection criterion during at least one half-period (14, 15) to determine whether it is the received signal caused by the step-like signal (10) and
if the check is positive, the signal propagation time or the change in the signal propagation time is determined with the help of an interpolated or extrapolated point of contact (20, 21) of the vibrational received signal (12) with the quiescent level (11) in a received signal/time diagram, and the signal propagation time and/or the change in the signal propagation time is/are evaluated on the basis of saved information as a measure of the blood composition and/or the change in the blood composition, wherein the area enclosed between the vibrational received signal (12) and the quiescent level (11) is determined during the half-period (14), and the area thereby ascertained is compared with a comparative value as a selection criterion, or the period of time of one or more half-periods (14, 15) of the vibrational received signal (12) is determined as the selection criterion and then is compared with a comparative value.

2. The method according to Claim 1, **characterized in that** the point (21) in the received signal/time diagram at which the vibrational received signal (12) deviates from the quiescent level (11) at the start of the first half-period (14) is determined as being the interpolated or extrapolated point of contact, such that the signal propagation time is derived from the point in time thereby ascertained.

3. The method according to Claim 1, **characterized in that** the point (20) in the received signal/time diagram at which the vibrational received signal (12) intersects the quiescent level (11) after the first half-period (14) is determined as the interpolated or extrapolated point of contact, such that the change in the signal propagation time is derived from the point in time thereby ascertained.

4. The method according to any one of the preceding claims, **characterized in that** the area enclosed between the vibrational received signal (12) and the quiescent level (11) is determined during the half-period (14); the subsequent half-period (15) is also scanned and detected, and the area enclosed between the vibrational received signal (12) and the quiescent level (11) is determined during the subsequent half-period (15); and the area enclosed between the vibrational received signal (12) and the quiescent level (11) during the subsequent half-period (15) is compared with a comparative value as the selection criterion.

5. The method according to any one of Claims 1 through 3, **characterized in that** the extreme value (18) of the vibrational received signal (12) during the half-period (14) is determined and compared with a comparative value.

6. The method according to Claim 5, **characterized in that** the subsequent half-period (15) is also scanned and detected, and the extreme value (19) of the vibrational received signal (12) is determined during the subsequent half-period (15) as an additional selection criterion and is compared with a comparative value.

7. The method according to any one of the preceding claims, **characterized in that** the quiescent level (11) is determined as the average of the half-periods (14) of the preceding scanned received signal values (13).

8. The method according to Claim 4, **characterized in that** the areas determined are evaluated as a measure of the damping of the signal.

9. A device for use of the method according to any one of Claims 1 to 8, comprising
an ultrasonic transmitter (2) for emitting the step-like signal (10),
an ultrasonic receiver (3) arranged at a distance from the ultrasonic transmitter (2) separated by the measuring zone, said ultrasonic receiver serving to deliver a received signal (12) that vibrates about a quiescent level (11) as a response to the step-like signal (10) propagation through the measuring zone,
a line carrying blood (1) in the measuring zone, an evaluation unit (6) connected to the ultrasonic transmitter (2) and the ultrasonic receiver (3),
wherein the evaluation unit (6) receives signals synchronous to the sending of the transmission signal and has a sampling device for sampling and saving the vibrational received signal (12) at regular intervals of time Δt,
wherein the evaluation unit (6) is also equipped to check on the vibrational received signal (12) on the basis of a selection criterion during a half-period (14, 15) to determine whether it is the received signal created by the step-like signal (10), wherein the area enclosed between the vibrational received signal (12) and the quiescent level (11) is determined during the half-period (14), and the area thereby ascertained is compared with a comparative value as a selection criterion, or the period of time of one or more half-periods (14, 15) of the vibrational received signal (12) is determined as the selection criterion and is compared with a comparative value, and
if the check is positive, the signal propagation time or the change in the signal propagation time is to be determined with the help of an interpolated or extrapolated point of contact (20, 21) of the vibrational received signal (12) with the quiescent level (11) in a received signal/time diagram and wherein the evaluation unit (6) is also suitable for evaluating the signal propagation time and/or the change in the signal propagation time as a measure of the blood composition and/or the change in the blood composition on the basis of saved information.

10. The device according to Claim 9, **characterized in that** it is a blood volume sensor.

11. The device according to Claim 10, **characterized in that** it is also an air detection sensor.

## Revendications

1. Procédé de mesure d'une composition, respectivement, d'une modification de composition de sang par la mesure d'un temps de propagation du signal dans le sang qui nécessite un signal pour effectuer un trajet de mesure d'un émetteur d'ultrasons (2) à un récepteur d'ultrasons (3), sachant qu'un tube conduisant le sang se trouve dans le trajet de mesure, ou de modifications de ce temps de propagation du signal, sachant
qu'un signal de type progressif (10) est émis avec l'émetteur d'ultrasons (2) et
que le signal de type progressif (10), après exécution du trajet de mesure, conduit à un signal de réception de type ondulatoire (12) sur un seuil de repos (11) sur le récepteur d'ultrasons (3) qui est échantillonné et détecté à intervalles réguliers Δt,
que le signal de réception de type ondulatoire (12) est vérifié à ce sujet à l'aide d'un critère de sélection pendant une demi-période (14, 15) afin de savoir s'il s'agit du signal de réception provoqué par le signal de type progressif (10) et
que si la vérification est positive, le temps de propagation du signal ou la modification du temps de propagation du signal est calculé(e) à l'aide d'un point de contact interpolé ou extrapolé (20, 21) du signal de réception de type ondulatoire (12) avec le seuil de repos (11) dans un diagramme temporel de signal de réception, et le temps de propagation du signal, respectivement, la modification du temps de propagation du signal est évalué (e) en tant que mesure de la composition, respectivement, de la modification de composition du sang sur la base des informations consignées, sachant que la surface incluse entre le signal de réception de type ondulatoire (12) et le seuil de repos (11) est calculée pendant la demi-période (14) et la surface ainsi calculée est comparée à une valeur comparative en tant que critère de sélection ou bien la durée d'une ou plusieurs demi-période(s) (14, 15) du signal de réception de type ondulatoire (12) est/sont calculé(e)s et comparé(e)s à une valeur comparative en tant que critère de sélection.

2. Procédé selon la revendication 1, **caractérisé en ce qu'**en tant que point de contact interpolé ou extrapolé, le point (21) auquel le signal de réception de type ondulatoire (12) s'écarte du seuil de repos (11) au début de la première demi-période (14) est calculé dans le diagramme temporel de signal de réception, sachant que le temps de propagation du signal est calculé à partir du moment ainsi calculé.

3. Procédé selon la revendication 1, **caractérisé en ce qu'**en tant que point de contact interpolé ou extrapolé, le point (20) auquel le signal de réception de type ondulatoire (12) coupe le seuil de repos (11) après la première demi-période (14), est calculé dans le diagramme temporel de signal de réception, sachant que la modification du temps de propagation du signal est déduite du moment ainsi calculé.

4. Procédé selon l'une des revendications précédentes, **caractérisé en ce que** la surface incluse entre le signal de réception de type ondulatoire (12) et le seuil de repos (11) est calculée pendant la demi-période (14), que la demi-période (15) consécutive est également échantillonnée, détectée et que la surface incluse entre le signal de réception de type ondulatoire (12) et le seuil de repos (11) est calculée pendant la demi-période (15) consécutive et que la surface incluse entre le signal de réception de type ondulatoire (12) et le seuil de repos (11) est comparée à une valeur comparative en tant que critère de sélection pendant la demi-période (15) consécutive.

5. Procédé selon l'une des revendications 1 à 3, **caractérisé en ce que** la valeur extrême (18) du signal de réception de type ondulatoire (12) est calculée pendant la demi-période (14) et comparée à une valeur comparative.

6. Procédé selon la revendication 5, **caractérisé en ce que** la demi-période (15) consécutive est également échantillonnée et détectée et qu'en tant que critère de sélection supplémentaire, la valeur extrême (19) du signal de réception de type ondulatoire (12) est calculée pendant la demi-période (15) et comparée à une valeur comparative.

7. Procédé selon l'une des revendications précédentes, **caractérisé en ce que** le seuil de repos (11) est calculé en tant que valeur moyenne à partir de valeurs de signal de réception (13) précédentes échantillonnées de la demi-période (14).

8. Procédé selon la revendication 4, **caractérisé en ce que** les surfaces calculées sont évaluées en tant que mesure de l'amortissement du signal.

9. Dispositif pour l'application du procédé selon l'une des revendications 1 à 8, comprenant
un émetteur d'ultrasons (2) pour émettre le signal de type progressif (10),
un récepteur d'ultrasons (3) à distance de l'émetteur d'ultrasons (2) par le trajet de mesure pour émettre un signal de réception de type ondulatoire (12) sur un seuil de repos (11) en réponse au signal de type progressif (10) traversant le trajet de mesure,
un tube (1) conduisant le sang se trouvant dans le trajet de mesure,
une unité d'évaluation (6) reliée à l'émetteur d'ultrasons (2) et au récepteur d'ultrasons (3),
sachant que l'unité d'évaluation (6) reçoit des signaux synchrones pour envoyer le signal d'émission et dispose d'un dispositif d'échantillonnage pour échantillonner et consigner le signal de réception de type ondulatoire (12) à intervalles réguliers Δt,
sachant que l'unité d'évaluation (6) est en outre équipée pour vérifier le signal de réception de type ondulatoire (12) à ce sujet à l'aide d'un critère de sélection au moins pendant une demi-période (14, 15) afin de savoir s'il s'agit du signal de réception provoqué par le signal de type progressif (10), sachant que la surface incluse entre le signal de réception de type ondulatoire (12) et le seuil de repos (11) est calculée pendant la demi-période (14) et la surface ainsi calculée est comparée à une valeur comparative en tant que critère de sélection ou bien la durée d'une ou plusieurs demi-période(s) (14, 15) du signal de réception de type ondulatoire (12) est/sont calculé(e)s et comparé(e)s à une valeur comparative en tant que critère de sélection et
que si la vérification est positive, le temps de propagation du signal ou la modification du temps de propagation du signal est calculé(e) à l'aide d'un point de contact interpolé ou extrapolé (20, 21) du signal de réception de type ondulatoire (12) avec le seuil de repos (11) dans un diagramme temporel de signal de réception, et sachant que l'unité d'évaluation (6) est en outre appropriée pour évaluer le temps de propagation du signal respectivement la modification du temps de propagation du signal en tant que mesure de la composition respectivement de la modification de composition du sang sur la base des informations consignées.

10. Dispositif selon la revendication 9, **caractérisé en ce qu'**il s'agit d'un capteur de volume sanguin.

11. Dispositif selon la revendication 10, **caractérisé en ce qu'**il s'agit en outre d'un capteur de détection d'air.
